# EUROPÄISCHE PATENTSCHRIFT

(11) **EP 3 010 390 B1**
(45) Veröffentlichungstag und Bekanntmachung des Hinweises auf die Patenterteilung: **07.06.2017**
(21) Anmeldenummer: 14729202.3
(22) Anmeldetag: 28.03.2014
(51) Int. Cl.: A61B 3/113, A61F 9/008

(54) **SYSTEM ZUM ERFASSEN VON POSITIONSDATEN WENIGSTENS EINES ELEMENTES IM VORDEREN BEREICH EINES AUGES**
SYSTEM FOR DETECTING POSITION DATA OF AT LEAST ONE ELEMENT IN THE FRONT REGION OF AN EYE
SYSTÈME DE DÉTECTION DE DONNÉES DE POSITION D'AU MOINS UN ÉLÉMENT DANS LA RÉGION ANTÉRIEURE D'UN OEIL

(30) Priorität: 19.06.2013 DE 102013106420
(43) Veröffentlichungstag der Anmeldung: 27.04.2016
(73) Patentinhaber: Heidelberg Engineering GmbH, 69121 Heidelberg (DE)
(72) Erfinder: ENGELHARDT, Ralf, 23568 Lübeck (DE); MARTENSEN, Björn, 23560 Lübeck (DE)
(74) Vertreter: Angerhausen, Christoph
(86) Internationale Anmeldenummer: PCT/DE2014/100108
(87) Internationale Veröffentlichungsnummer: WO 2014/202047

(56) Entgegenhaltungen:
- EP-A1- 1 985 269
- WO-A1-93/16631
- US-A- 4 848 340
- US-A1- 2008 055 543
- US-A1- 2011 149 241

## Beschreibung

Die Erfindung betrifft ein System zum Erfassen von Positionsdaten wenigstens eines Elementes im vorderen Bereich eines Auges, wobei das System zumindest eine erste Systemkomponente und eine zweite Systemkomponente aufweist. Die EP 1 985 269 A1 beschreibt ein derartiges System, bei dem die erste Systemkomponente zum kontinuierlichen Erfassen von Positionsänderungen der Vertex der Hornhaut des Auges ausgelegt ist, die zweite Systemkomponente elektromagnetische Strahlung auf zu ermittelnde oder zu verändernde topographische Strukturen der Hornhaut lenkt, und wobei es eine Steuerungseinrichtung mit Prozessor und Speicher aufweist, wobei die Ausrichtung der ersten Systemkomponente in Bezug auf die Ausrichtung der zweiten Systemkomponente definiert ist, und wobei die zweite Systemkomponente kontinuierlich anhand der von der ersten Systemkomponente erfassten Positionsänderungen von der Steuerungseinrichtung auf ihre jeweilige Sollposition geführt wird. Ähnliche Systeme beschreiben die US 2011/0149241 A1 und die US 4,848,340 A. Ein ähnliches System ist auch aus der DE 10 2009 030 466 A1 bekannt. Dort werden in Vorbereitung einer refraktiven Laserbehandlung des Auges zunächst die aktuellen Daten von physikalischen Einflussgrößen auf die Abbildung des Auges erhoben und im Folgenden die zu korrigierenden Augenfehler bestimmt. Diese Korrekturen für den Abbildungsprozess des Auges müssen anschließend in eine Behandlungsprozedur umgewandelt werden, was beispielsweise zu einem lokalen Abtrag von Hornhautgewebe zum Erzeugen einer entsprechend angepassten Hornhautform führt. Für eine richtige Korrektur ist es dabei unabdingbar, dass sich die Diagnosedaten und die Therapiedaten auf einen gemeinsamen geometrischen Referenzpunkt beziehen. Gemäß der DE 10 2009 030 466 A1 ist als Referenzpunkt der Vertex einer Hornhaut als vorteilhaft erkannt worden, also der Ort der höchsten Erhebung der Oberfläche der Hornhaut, auf den die ermittelten Datensätze registriert werden.

Bei der Untersuchung beziehungsweise der Behandlung des vorderen Auges mittels zeitlich seriell erfolgender Diagnose- und/oder Behandlungsabläufe, die in der Regel aus mehreren Schritten bestehen, wird durch die Okulomotorik, sei diese willkürlich oder unwillkürlich, bewusst oder unbewusst, die Integrität und Kontinuität des Vorgangs beeinträchtigt. Nachträgliche Korrekturen und/oder Registrierungen sind für Diagnosedaten zwar möglich, enthalten bei starken Bewegungen jedoch inhomogene Informationsdichten, welche zu lokalen Ungenauigkeiten führen. Für Behandlungssysteme ist eine nachträgliche Korrektur nicht praktikabel.

Es ist daher die Aufgabe der Erfindung, ein System für die Erfassung von Positionsdaten wenigstens eines Elementes im vorderen Bereich eines Auges zur Verfügung zu stellen, das die kontinuierliche Adaption auf Augenbewegungen für die zuverlässige Erzeugung von Diagnosedatensätzen und/oder Datensätze für Materialveränderungen gewährleistet.

Diese Aufgabe wird durch ein System nach Anspruch 1 gelöst. Vorteilhafte Ausgestaltungen sind Gegenstand der jeweils rückbezogenen Unteransprüche.

Mit dem erfindungsgemäßen System ist ein kontinuierliches Erfassen von Positionsänderungen zumindest eines ausgezeichneten Ortes und/oder zumindest einer ausgezeichneten Struktur auf der Hornhaut und/oder dem hinter der Hornhaut liegenden Bereich eines Auges möglich, indem die erste Systemkomponente auf diesen beziehungsweise auf diese ausgerichtet wird; weiter ein Lenken einer elektromagnetischen Strahlung auf zu ermittelnde oder zu verändernde topographische Strukturen der Hornhaut durch Ausrichtung der zweiten Systemkomponente, wobei die Ausrichtung der ersten Systemkomponente in Bezug auf die Ausrichtung der zweiten Systemkomponente definiert ist und wobei die zweite Systemkomponente kontinuierlich anhand der von der ersten Systemkomponente erfassten Positionsänderungen auf ihre jeweilige Sollposition geführt wird. Die erfassten Informationen ermöglichen das kontinuierliche Nachführen oder Tracking des Diagnose- und/oder Behandlungssystems.

Weiterhin kann einer der ausgezeichneten Orte der Vertex der Hornhaut sein, und es werden somit die Positionsänderungen des Vertex erfasst. Die berechnete Vertexposition enthält Informationen über die Translation des gesamten Augapfels im Anwendungsgebiet, wie sie beispielsweise durch die unvollständige Fixierung des Kopfes entstehen. Werden zusätzlich weitere Strukturen des Auges ausgewertet, wie es weiter unten noch beschrieben wird, können weitere Freiheitsgrade bestimmt werden.

Die Erfassung der Vertexposition ist bereits aus der DE 10 2009 030 466 A1 bekannt. Hierbei kann aber nun vorgesehen sein, dass die Hornhaut mit mindestens einer Lichtquelle beleuchtet wird, so dass Reflexionen auf der Hornhaut entstehen, wobei dann die aktuelle Position des Vertex aus der Position der Reflexionen bestimmt wird.

Die Positionsänderung der Reflexionen kann unmittelbar zum Führen der zweiten Systemkomponente auf ihre jeweilige Sollposition verwendet werden.

Andererseits hat es aber auch Vorteile, wenn mehrere Lichtquellen symmetrisch um die Hauptachse des Systems angeordnet werden. Dann nämlich können die Positionen der durch die Lichtquellen auf dem Auge hervorgerufenen Reflexionspunkte zum Bestimmen einer zumindest angenäherten Position des Vertex verwendet werden. Bei einer symmetrischen Anordnung der Lichtquellen um die Hauptachse des Systems herum entsteht nämlich ein symmetrisches Muster aus Reflexionspunkten, wobei ein Symmetrieursprung der Anordnung der Reflexionspunkte zumindest näherungsweise der Position des Vertex entspricht. Dabei kann der Symmetrieursprung mit Hilfe einer Konvolution bestimmt werden. Ein solches Verfahren ist sehr schnell und ohne großen Aufwand realisiert.

Der Vertex kann somit über die Reflexionspunkte hinreichend genau bestimmt werden. Es entsteht allerdings ein Parallaxenfehler, wenn der Vertex nicht auf der optischen Hauptachse liegt. Dieser Parallaxenfehler kann korrigiert werden, wenn der Radius der Wölbung der Hornhaut bekannt ist: Menschliche Hornhäute variieren im Wölbungsradius nicht sehr stark, sie zeigen Werte zwischen 7 mm und 9 mm, so dass die Parallaxenkorrektur relativ gut ist, wenn sie für eine Kugel mit Durchmesser 8 mm bestimmt wird.

Um eine solche Vorgehensweise zu realisieren, kann vorteilhaft weiter vorgesehen sein, dass die Position eines weiteren der ausgezeichneten Orte oder einer weiteren der ausgezeichneten Strukturen auf der Hornhaut und/oder dem hinter der Hornhaut liegenden Bereich eines Auges relativ zum Vertex bestimmt wird, wobei insbesondere vorgesehen ist, dass die weitere ausgezeichnete Struktur die Pupille des Auges ist.

Dabei kann/können auch zur Bestimmung der Geometrie der Pupille die Position der Reflexion oder die Positionen der Reflexionen einbezogen werden.

Weiter können aus Positionsänderungen des weiteren ausgezeichneten Ortes und/oder der weiteren ausgezeichneten Struktur auf der Hornhaut und/oder dem hinter der Hornhaut liegenden Bereich eines Auges relativ zum Vertex rotatorische Lageänderungen - auch als Cyclotorsion bezeichnet - des Auges ermittelt werden. Durch Auswertung der Pupillenposition in Bezug auf die Vertexposition wird die Verkippung des Augapfels und die Fixationsfähigkeit während eines Diagnose- und/oder Behandlungsvorgangs ermittelt.

Dies bedeutet, dass anhand der rotatorischen Lageänderungen des Auges die Fixation überwacht werden kann, so dass gegebenenfalls ein Fixationsverlust festgestellt wird. Mit Fixation wird in der Augenheilkunde das gezielte Betrachten eines Objektes im Außenraum bezeichnet, was im Normalfall mit der Netzhautstelle höchster Auflösung, der Fovea centralis, geschieht. Hierbei vermittelt dieser Punkt das Richtungsempfinden "geradeaus" und repräsentiert damit die physiologische Hauptsehrichtung des Auges.

Es kann vorgesehen sein, dass anhand der Qualität der Reflexionspunkte die Güte der Hornhautoberfläche und/oder die Beschaffenheit des Tränenfilms auf der Hornhautoberfläche ermittelt werden. Zwar ist das Tracking, wenn es gemäß der vorliegenden Erfindung durchgeführt wird, von vornherein relativ genau und robust, es kann jedoch zu kleineren Sprüngen beispielsweise bei Abbruch des Tränenfilms führen. Diesen Ungenauigkeiten kann durch eine Qualitätsüberprüfung vorgebeugt werden.

Mit der zweiten Systemkomponente können nun ein- oder mehrdimensionale Tiefenschnittbilder aufgenommen werden. Diese haben eine bekannte Position relativ zum Vertex.

Dabei wird der Vertex anhand des aktuellen Kamerabildes jeweils neu bestimmt. Die Bewegung im Kamerabild kann anhand der Reflexionspunkte relativ zum ersten Kamerabild bestimmt werden, so dass eine Verrechnung in die Scannerkoordinaten erfolgen kann. Die Bewegung im Kamerabild wird anhand sonstiger Strukturen im ganzen Bild in einem oder in mehreren Ausschnitten relativ zum ersten Kamerabild bestimmt und in die Scannerkoordinaten verrechnet.

Somit können jetzt beliebige Scanmuster, wie Raster oder Meridiane, aufgenommen werden. Insbesondere ist sichergestellt, dass meridiane Schnitte durch den Vertex gehen und eine Refraktionskorrektur in einer Ebene erfolgen kann. Topographien können ohne nachträgliche laterale Bewegungsregistrierungen aufgenommen werden. Axiale Bewegungen können einfach ausgerechnet werden, da insbesondere bei meridianen Scanmustern alle Tiefenschnitte eine gemeinsamen Punkt haben.

Deswegen ist vorgesehen, dass axiale Positionsänderungen aus Unterschieden zwischen an derselben Position in der Geometrie des Auges auf der Frontalebene aufgenommenen Tiefenschnitten bestimmt werden. Jederzeit kann die Position des Vertex anhand eines an dieser Position aufgenommenen Tiefenschnittbildes überprüft werden.

Als Alternative kann eine dritte Systemkomponente zu diesem Zweck vorgesehen sein, wobei die Ausrichtung der ersten und der zweiten Systemkomponente relativ zueinander und zur dritten Systemkomponente definiert ist und die Positionierung der dritten Systemkomponente kontinuierlich anhand der von der ersten und der zweiten Systemkomponente erfassten Positionsänderungen auf die jeweilige Sollposition geregelt wird.

Unter Verwendung der ermittelten Positionsinformation kann die Aktivierung und die Deaktivierung der zweiten und gegebenenfalls dritten Systemkomponente gesteuert werden. Auch können die Aufnahme beziehungsweise das Verwerfen von Messwerten gesteuert werden.

Im Folgenden soll die Erfindung anhand der beigefügten Zeichnung näher erläutert werden. Es zeigt:
- Figur 1: eine schematische Ansicht eines Auges, wobei auf der Hornhaut Reflexionspunkte durch externe Lichtquellen erzeugt worden sind;
- Figur 2: eine eindimensionale Darstellung der Reflexionspunkte im Schnitt; und
- Figur 3: die Konvolution des Bildes aus Figur 2 mit dem invertierten Bild.

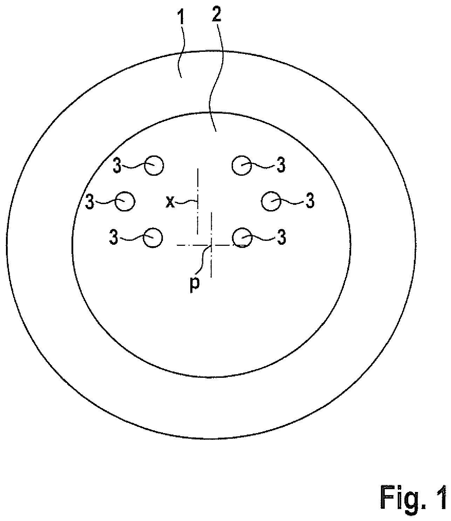

Figur 1 zeigt eine schematische Darstellung des vorderen Bereiches eines menschlichen Auges, bei dem eine Pupille 2 von einer Iris 1 umgeben ist. Mit p ist der Pupillenmittelpunkt angegeben. Bei einem erfindungsgemäßen System sind Lichtquellen um die Hauptachse des Diagnose- und/oder Behandlungssystems symmetrisch angeordnet, was zu einem Muster aus Reflexionspunkten 3 führt. Der Mittelpunkt x der Reflexionspunkte 3 entspricht in Näherung dem Vertex des Auges. Die Position des Vertex des Auges ist unter Anderem abhängig von der Fixation und der Fixationsachse. Wird die Position der Pupille 2 oder des Pupillenmittelpunkts p in Bezug auf den Mittelpunkt x der Reflexionspunkte 3 verfolgt, kann die Fixation überwacht und ein Fixationsverlust, welcher zur Fehlausrichtung des Auges führt, detektiert werden.

Insbesondere für Systeme, welche geometrische Information des vorderen Auges sequentiell in einem Zeitintervall, beispielsweise über einzelne Tiefschnitte, ermitteln, ist diese Überwachung sinnvoll, da hierdurch Informationen über die Ausrichtung des Auges in der Sequenz vorliegen. Die Bestimmung der Vertexposition des vorderen Auges kann auch für komplexe Geometrien der die Reflexionspunkte erzeugenden Lichtquellen am Diagnose- und/oder Behandlungssystem durch Strahlverfolgungen erfolgen, wie sie auch für die Generierung von 3D-Computergrafiken in großem Umfang durchgeführt werden. Eine Darstellung findet sich im Reflections on Spheres and Cylinders of Revolution, Georg Glaeser, Journal for Geometry and Graphics, Volume 3 (1999), No. 2, 121-139*.*

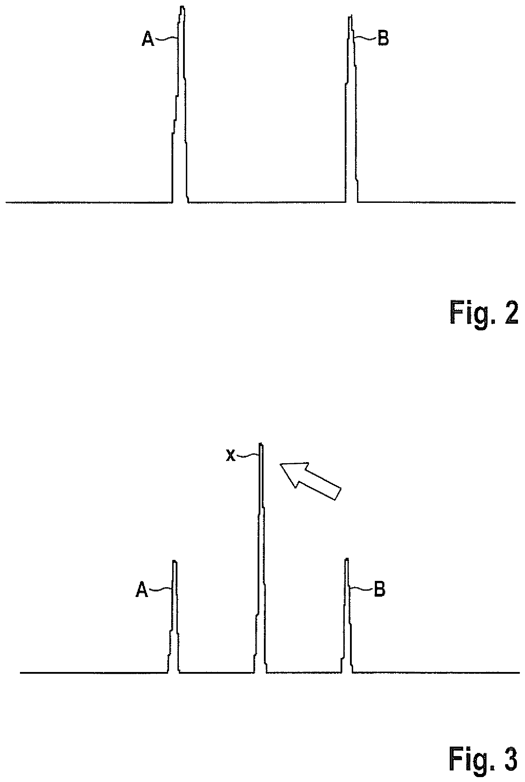

Figur 2 zeigt eine eindimensionale Darstellung der Reflexionspunkte im Schnitt, und zwar am Beispiel der Signalspitzen A und B. Es versteht sich, dass weitere Reflexionspunkte durch ähnliche Signalspitzen abzubilden sind. Durch die Konvolution des Bildes der Figur 2 mit dem invertierten Bild wird, wie in Figur 3 gezeigt, der Mittelpunkt x der Reflexionspunkte 3 (Figur 1) ermittelt, der in Näherung dem Vertex der Hornhaut beziehungsweise des Auges entspricht.

Die in der vorstehenden Beschreibung, in den Zeichnungen sowie in den Ansprüchen offenbarten Merkmale der Erfindung können sowohl einzeln als auch in beliebiger Kombination für die Verwirklichung der Erfindung wesentlich sein.

## Patentansprüche

1. System zum Erfassen von Positionsdaten wenigstens eines Elementes im vorderen Bereich eines Auges, wobei das System zumindest eine erste Systemkomponente und eine zweite Systemkomponente aufweist, wobei
- die erste Systemkomponente zum kontinuierlichen Erfassen von Positionsänderungen der Vertex der Hornhaut des Auges ausgelegt ist;
- die zweite Systemkomponente elektromagnetische Strahlung auf zu ermittelnde oder zu verändernde topographische Strukturen der Hornhaut lenkt; und
- wobei das System eine Steuerungseinrichtung mit Prozessor und Speicher aufweist;
wobei die Ausrichtung der ersten Systemkomponente in Bezug auf die Ausrichtung der zweiten Systemkomponente definiert ist und wobei die zweite Systemkomponente kontinuierlich anhand der von der ersten Systemkomponente erfassten Positionsänderungen von der Steuerungseinrichtung auf ihre jeweilige Sollposition geführt wird, **dadurch gekennzeichnet, dass** Lichtquellen, welche die Hornhaut beleuchten, symmetrisch um die Hauptachse des Systems angeordnet sind, so dass ein Muster aus Reflexionspunkten (3) auf der Hornhaut entsteht, wobei der Prozessor die aktuelle Position des Vertex als den Mittelpunkt (x) der Reflexionspunkte (3) bestimmt.

2. System nach Anspruch 1, **dadurch gekennzeichnet, dass** eine dritte Systemkomponente vorhanden ist, deren Ausrichtung zur ersten und zur zweiten Systemkomponente definiert ist und die von der Steuerungseinrichtung anhand erfasster Positionsänderungen auf ihre jeweilige Sollposition zum Verändern von körpereigenem und/oder implantierten Material geführt wird.

3. System nach Anspruch 1, **dadurch gekennzeichnet, dass** mehrere Lichtquellen symmetrisch um die Hauptachse des Systems angeordnet sind.

4. System nach Anspruch 1, **dadurch gekennzeichnet, dass** ein Ursprungspunkt der Anordnung der Reflexionspunkte zumindest näherungsweise der Position des Vertex entspricht.

5. System nach Anspruch 4, **dadurch gekennzeichnet, dass** der Ursprungspunkt mit Hilfe einer Konvolution bestimmt ist.

6. System nach einem der Ansprüche 1 bis 5, **dadurch gekennzeichnet, dass** die Position eines weiteren ausgezeichneten Ortes und/oder einer weiteren ausgezeichneten Struktur auf der Hornhaut und/oder dem hinter der Hornhaut liegenden Bereich eines Auges relativ zum Vertex bestimmt ist.

7. System nach Anspruch 6, **dadurch gekennzeichnet, dass** die weitere ausgezeichnete Struktur die Pupille des Auges ist.

8. System nach Anspruch 7, **dadurch gekennzeichnet, dass** die Geometrie der Pupille aus der Position der Reflexion oder den Positionen der Reflexionen abgeleitet wird.

9. System nach einem der Ansprüche 5 bis 8, **dadurch gekennzeichnet, dass** aus Positionsänderungen des weiteren ausgezeichneten Ortes und/oder der weiteren ausgezeichneten Struktur auf der Hornhaut und/oder dem hinter der Hornhaut liegenden Bereich eines Auges relativ zum Vertex rotatorische Lageänderungen des Auges ermittelt sind.

10. System nach Anspruch 9, **dadurch gekennzeichnet, dass** anhand der rotatorischen Lageänderungen des Auges die Fixation überwacht und gegebenenfalls ein Fixationsverlust festgestellt sind.

11. System nach einem der vorangegangenen Ansprüche, bei dem anhand der Qualität der Reflexionspunkte die Güte der Hornhautoberfläche und/oder die Beschaffenheit des Tränenfilms auf der Hornhautoberfläche ermittelt ist.

12. System nach Anspruch 1, **dadurch gekennzeichnet, dass** mit der zweiten Systemkomponente ein- oder mehrdimensionale Tiefenschnittbilder aufgenommen sind.

13. System nach Anspruch 12, **dadurch gekennzeichnet, dass** axiale Positionsänderungen aus Unterschieden zwischen an derselben Position in der Geometrie des Auges auf der Frontalebene aufgenommenen Tiefschnitten bestimmt werden.

14. System nach einem der vorangegangenen Ansprüche, **dadurch gekennzeichnet, dass** die Position des Vertex anhand eines an dieser Position aufgenommenen Tiefenschnittbildes überprüft ist.

## Claims

1. A system for detecting position data of at least one element in the front region of an eye, the system comprising at least one first system component and a second system component, wherein
- the first system component is designed to continuously detect position changes of the vertex of the cornea of the eye;
- the second system component directs electromagnetic radiation at topographical structures of the cornea to be ascertained or to be modified; and
- wherein the system comprises a control unit including a processor and a memory;
wherein the alignment of the first system component with respect to the alignment of the second system component being defined, and the second system component being continuously guided to the respective target position thereof by the control unit based on the position changes detected by the first system component,
**characterised in that** light sources, which illuminate the cornea, are symmetrically disposed around the main axis of the system, so that a pattern of reflection points (3) is created on the cornea, the processor determining the current position of the vertex as the center (x) of the reflection points (3).

2. The system according to claim 1, wherein a third system component is provided, the alignment of which with respect to the first and second system components is defined, and which is guided by the control unit to the respective target position thereof based on detected position changes to modify endogenous and/or implanted material.

3. The system according to claim 1, wherein multiple light sources are disposed symmetrically around the main axis of the system.

4. The system according to claim 1, wherein a point of origin of the arrangement of the reflection points corresponds at least approximately to the position of the vertex.

5. The system according to claim 4, wherein the point of origin is determined with the aid of a convolution.

6. The system according to one of claims 1 to 5, wherein the position of a further distinct location and/or of a further distinct structure on the cornea and/or on the region of an eye located behind the cornea relative to the vertex is determined.

7. The system according to claim 6, wherein the further distinct structure is the pupil of the eye.

8. The system according to claim 7, wherein the geometry of the pupil is derived from the position of the reflection or the positions of the reflections.

9. The system according to one of claims 5 to 8, wherein the position changes of the further distinct location and/or of the further distinct structure on the cornea and/or on the region of an eye located behind the cornea relative to the vertex are used to ascertain rotational position changes of the eye.

10. The system according to claim 9, wherein fixation is monitored based on the rotational position changes of the eye, and optionally a loss of fixation is noticed.

11. The system according to any one preceding claim, wherein the quality of the cornea surface and/or the condition of the precorneal film on the surface of the cornea is ascertained based on the quality of the reflection points.

12. The system according to claim 1, wherein the second system component is used to record one- or multi-dimensional deep sectional images.

13. The system according to claim 12, wherein axial position changes are determined from differences between deep sections recorded at the same position in the geometry of the eye on the frontal plane.

14. The system according to any one preceding claim, wherein the position of the vertex is checked based on a deep sectional image that is recorded at this position.

## Revendications

1. Système pour le relevé de données de position d'au moins un élément dans la zone avant d'un oeil, le système comprenant au moins un premier composant de système et un deuxième composant de système,
- le premier composant du système étant conçu pour le relevé en continu de changements de position du vertex de la cornée de l'oeil ;
- le deuxième composant du système déviant un rayonnement électromagnétique vers des structures topographiques à déterminer ou à modifier ; et
- le système comprenant un dispositif de commande avec un processeur et une mémoire ;
l'orientation du premier composant du système étant définie par rapport à l'orientation du deuxième composant du système et le deuxième composant du système étant guidé en continu par le dispositif de commande, à l'aide des changements de position relevés par le premier composant du système, vers sa position de consigne,
**caractérisé en ce que** des sources de lumière, qui éclairent la cornée, sont disposées symétriquement autour de l'axe principal du système, de façon à ce qu'une matrice de points de réflexion (3) apparaisse sur la cornée, le processeur déterminant la position actuelle du vertex en tant que point central (x) des points de réflexion (3).

2. Système selon la revendication 1, **caractérisé en ce qu'**un troisième composant du système est présent, dont l'orientation est définie par rapport au premier et au deuxième composant du système et qui est guidé, par le dispositif de commande à l'aide de changements de position relevés, vers sa position de consigne pour la modification d'un matériau propre au corps et/ou implanté.

3. Système selon la revendication 1, **caractérisé en ce que** plusieurs sources de lumière sont disposées symétriquement autour de l'axe principale du système.

4. Système selon la revendication 1, **caractérisé en ce qu'**un point d'origine de la disposition des points de réflexion correspond au moins approximativement à la position du vertex.

5. Système selon la revendication 4, **caractérisé en ce que** le point d'origine est déterminé à l'aide d'une convolution.

6. Système selon l'une des revendications 1 à 5, **caractérisé en ce que** la position d'un autre endroit distingué et/ou d'une autre structure distinguée sur la cornée et/ou la zone se trouvant derrière la cornée d'un oeil est déterminée par rapport au vertex.

7. Système selon la revendication 6, **caractérisé en ce que** l'autre structure distinguée est la pupille de l'oeil.

8. Système selon la revendication 7, **caractérisé en ce que** la géométrie de la pupille est dérivée de la position de la réflexion ou des positions des réflexions.

9. Système selon l'une des revendications 5 à 8, **caractérisé en ce que**, à partir de changements de position de l'autre endroit distingué et/ou de l'autre structure distinguée sur la cornée et/ou la zone se trouvant derrière la cornée d'un oeil sont déterminés des changements de position rotatifs de l'oeil par rapport au vertex.

10. Système selon la revendication 9, **caractérisé en ce que**, à l'aide des changements de position rotatifs de l'oeil, la fixation est surveillée et, le cas échéant, une perte de fixation est constatée.

11. Système selon l'une des revendications précédentes, dans lequel, à l'aide de la qualité des points de réflexion, la qualité de la surface de la cornée et/ou l'état du film de larmes sur la surface de la cornée sont déterminées.

12. Système selon la revendication 1, **caractérisé en ce que**, avec le deuxième composant du système, sont enregistrées des images de coupe en profondeur monodimensionnelles ou pluridimensionnelles.

13. Système selon la revendication 12, **caractérisé en ce que** les changements de position axiaux sont déterminés à partir de différences entre des coupes en profondeur enregistrées à la même position dans la géométrie de l'oeil sur le plan frontal.

14. Système selon l'une des revendications précédentes, **caractérisé en ce que** la position du vertex est contrôlée à l'aide d'une image de coupe en profondeur enregistrée à cette position.
